(19) Europäisches Patentamt
European Patent Office
Office européen des brevets

(11) EP 0 517 326 B1

(12) **EUROPEAN PATENT SPECIFICATION**

(45) Date of publication and mention of the grant of the patent:
**16.08.2001 Bulletin 2001/33**

(51) Int Cl.[7]: **G01N 33/94**, G01N 33/532

(21) Application number: **92201581.3**

(22) Date of filing: **02.06.1992**

(54) **Labeled drug hapten analogues for immunoassays**

Markierte Hapten-Analoge zur Verwendung in Immunoassays

Analogues d'haptènes marqués pour utilisation dans des essais immunologiques

(84) Designated Contracting States:
**AT BE CH DE ES FR GB GR IT LI LU NL SE**

(30) Priority: **07.06.1991 US 712330**
**16.03.1992 US 851439**

(43) Date of publication of application:
**09.12.1992 Bulletin 1992/50**

(73) Proprietor: **Johnson & Johnson Clinical Diagnostics, Inc.**
**Rochester New York 14650 (US)**

(72) Inventors:
- **Danielson, Susan Jean,**
  **c/o EASTMAN KODAK COMPANY**
  **Rochester, New York 14650-2201 (US)**
- **Brummond, Barbara A.,**
  **c/o EASTMAN KODAK COMPANY**
  **Rochester, New York 14650-2201 (US)**
- **Oenick, Marsha D.,**
  **c/o EASTMAN KODAK COMPANY**
  **Rochester, New York 14650-2201 (US)**
- **Ponticello, Ignazio S.,**
  **c/o EASTMAN KODAK COMPANY**
  **Rochester, New York 14650-2201 (US)**
- **Hilborn, David Alan,**
  **c/o EASTMAN KODAK COMPANY**
  **Rochester, New York 14650-2201 (US)**

(74) Representative: **Mercer, Christopher Paul et al**
**Carpmaels & Ransford**
**43, Bloomsbury Square**
**London WC1A 2RA (GB)**

(56) References cited:
**EP-A- 0 233 690** **EP-A- 0 323 692**
**DE-A- 3 205 506** **GB-A- 2 039 485**
**US-A- 4 244 939**

- **JOURNAL OF IMMUNOASSAY vol. 4, no. 3, 1983, pages 209 - 327 ISHIKAWA ET AL. 'Enzyme -labeling of antibodies and their fragments for enzyme immunoassay and immunohistochemical staining'**
- **CLINICAL CHEMISTRY. vol. 28, no. 11, November 1982, WINSTON US pages 2278 - 2282 LU-STEFFES ET AL. 'Fluorescence polarization immunoassay IV. Determination of phenytoin and phenobarbital in human serum and plasma'**

Remarks:
The file contains technical information submitted after the application was filed and not included in this specification

EP 0 517 326 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

This invention relates to clinical chemistry particularly immunoassays.

Immunoassays, which take advantage of natural immunological reactions, have found wide-spread use as analytical techniques in clinical chemistry. Because of the specificity of the reactions, they are particularly advantageous in quantifying biological analytes that are present in very low concentration in biological fluids. Such analytes include, for example, antibodies, therapeutic drugs, narcotics, enzymes, hormones, proteins, and so forth.

In competitive binding immunoassays, a labeled ligand, including immunocompetent derivatives and analogs of the ligand, is placed in competition with unlabeled ligand for reaction with a fixed amount of the appropriate binding material (called a receptor herein). Unknown concentrations of the ligand can be determined from the measured signal of either the bound or unbound (that is, free) labeled ligand. The reaction proceeds as follows:

$$\text{ligand + labeled ligand + receptor} \rightarrow$$

$$\text{ligand-receptor + labeled ligand-receptor.}$$

Conventional labels include radioactive tags, enzymes, chromophores, fluorophores, stable free radicals, and enzyme cofactors, inhibitors and allosteric effectors.

Consistent with the foregoing an immunoassay for drug derivatives, such as phenobarbital and phenytoin, in serum can be based on competition of an enzyme labeled analogue of such drugs with the drug in the patient serum for immobilized antibody binding sites.

Specific requirements for the labeled drug hapten analogues(hereafter sometimes LDH) include: 1) at least 65% of the LDH can be bound by excess immobilized antibody; 2) affinity of the LDH for immobilized antibody is such that competition of a fixed amount of LDH with the drug occurs in a therapeutically relevant drug concentration range; and 3) stability of the LDH against hydrolysis of its enzyme label under storage conditions.

Requirements imposed on the drug hapten analogue include: 1) accessibility of the analogue to the immobilized antibody following conjugation with the enzyme label; 2) specific recognition of the labeled analogue by the antibody to the drug; and 3) sufficient reactivity of the analogue with the enzyme label, either directly or following activation of the enzyme or the analogue, under conditions that do not adversely affect enzyme activity.

Glucose oxidase (GOD) and alkaline phosphatase (ALP) enzyme labels coupled to phenobarbital and phenytoin hapten analogues disclosed in U.S. Patent 4,752,568 gave adequate enzyme labeled analogues for conducting effective competitive immunoassays in the desired format.

The problem is that the labeled phenobarbital and phenytoin analogues disclosed in the above patent were unsatisfactory for conducting competitive immunoassays when the enzyme horseradish peroxidase (HRP) was used as the label. The coupling reactions between such analogues and HRP were both slow and incomplete. Moreover phenobarbital and phenytoin HRP labels were bound very weakly so that much higher concentrations of label or antibody binding sites would be required to give a readable signal.

U.S. Patent 4,244,939 discloses compounds useful as tracers in radioimmunoassay of barbiturates in which a radioiodinated group is linked to a pyrimidine ring nitrogen atom of the barbiturate.

EP patent specification 0233690 describes labelled hydantoin conjugate and its use in analytical elements and immunoassays. The label is linked to the 3-nitrogen position of the hydantoin ring with a linkage derived from an aliphatic monocarboxylic acid having from 2 to 12 carbon atoms.

DE patent specification 3205506 describes a fluorescent label covalently linked to haptens.

The present invention provides labelled drug analogue having:

(A) an enzyme label;
(B) a drug nucleus selected from a hydantoin nucleus or a barbiturate nucleus; and
(C) a linking chain linking the 3-position of the drug hapten nucleus to the label through a carbonyl bridge wherein the linking chain, excluding the said carbonyl bridge, has 12 to 40 atoms consisting of:

(I) two or more $C_1$ to $C_{10}$ alkylene groups;
optionally in combination with
one or more phenylene groups, and/or
one or more 5 to 7 membered heterocyclic rings joined into the linking group through ring nitrogen atoms;
or
(II) one $C_1$ to $C_{10}$ alkylene group in combination with one or more phenylene groups and one or more 5 to 7 membered heterocyclic rings joined into the linking group through ring nitrogen atoms;

said groups and rings being bonded to each other through chemical groups selected from:

(a) esters, including thioesters, -COZ-, where Z is O or S;
(b) amides,-CONH-;
(c) hetero atoms selected from -O-, -S-, and -NR-; wherein R represents $C_1$ to $C_6$ alkyl (for example, methyl, ethyl, propyl, butyl, and so forth); and
(d) carbonyl.

The heterocyclic ring is preferably selected from a 1, 4-piperazinylene, 2, 5-dimethyl-1,4-piperazinylene; 1,3-imidazolidinylene, and 1,3-hexahydrodiazepinylene groups.
The new drug analogues (a) react with HRP, and other enzymes such as GOD and ALP, faster and more completely, than above mentioned prior art analogues (b) form covalent bonds with such enzymes without adverse effect on enzyme activity, and (c) the resulting labelled drug analogues are more readily recognized and tightly bound by antibodies to hydantoins and barbiturates.
The above labelled hydantoin and barbiturate derivatives include those that conform to the structure:

(I)

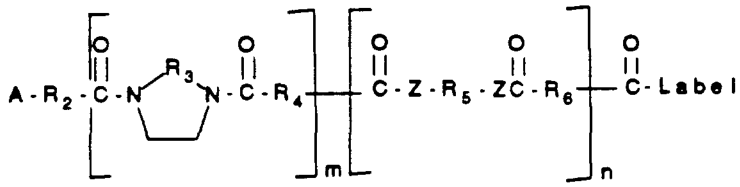

wherein

A represents a hydantoin nucleus of the structure

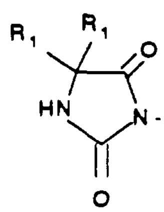

or a barbiturate nucleus of the structure

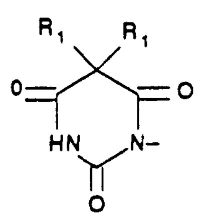

;

$R^1$ each independently represents hydrogen, alkyl of 1 to 10 carbon atoms, unsubstituted or substituted phenyl;
$R^2$, $R^4$, $R^5$, and $R^6$, each independently represents $C_1$ to $C_{10}$ alkylene or such alkylene groups interrupted with at least one or more ester groups, amide groups, -O-, -S-, or -NR-;
$R^3$ represents $C_1$ to $C_3$ alkylene;
Z represents -O-, -S-, and -NR-, wherein R represents hydrogen or $C_1$ to $C_6$ alkyl, for example, methyl propyl and hexyl;
m is 0, 1, or 2;

n is 0, 1, or 2;
m + n > 0; and
the total number of atoms comprised in m, n and $R^2$ is 12 to 40;
LABEL is an enzyme;
and further provided that (i) at least one of the $R^1$ groups is substituted or unsubstituted phenyl; (ii) one of $R^4$, $R^5$, and $R^6$ can be phenylene; and (iii) the bracketed components of structure I can appear therein in any order.

Consistent with the above definitions $R^1$ can represent hydrogen, methyl, propyl, hexyl, decyl, unsubstituted phenyl or phenyl substituted with alkyl of 1 to 6 carbon atoms, nitro, halogen, cyano, and alkoxy of 1 to 6 carbon atoms;

$R^2$, $R^4$, $R^5$, and $R^6$, can each independently represent alkylene selected from ethylene, butylene, pentylene, octylene, or such alkylene interrupted with at least one or more ester groups, amide groups, -O-,-S-, and -NR-;
$R^3$ represents methylene, ethylene, or trimethylene;
Z each independently represents -O-, -S-,or-NR-, wherein R represents at least one of hydrogen, methyl, propyl, or hexyl; and
LABEL represents an enzyme.

At least 65% of the labeled drug hapten analogues can be bound by excess immobilized barbiturate or hydantoin antibodies. The labeled analogues with extended linkers, particularly those having amide bonds in the linking chain are similarly bound by all the immobilized antibody types used in reducing this invention to practice. Also the derivatives having the amide in the extended linker are very stable against hydrolysis.

The labeled drug analogues were prepared from novel drug hapten analogues described *infra*. They generally comprise:

(a) an active ester group, such as a succinimidoxycarbonyl;
(b) a hydantoin or barbiturate nucleus, and
(c) a linking chain linking the active ester group to the hydantoin or barbiturate nucleus; wherein the linking chain is as previously defined.

More specifically, the preferred new hydantoin active ester are those conforming to the structure:

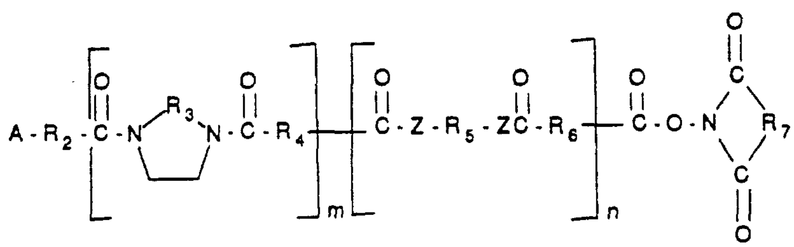

wherein

A, $R^1$, $R^2$, $R^3$, $R^4$, $R^5$, $R^6$, Z, m, and n and the provisos related thereto are as previously defined, and
$R^7$ is an ethylene or o-phenylene group. Hydantoin Drug Hapten Analogues

The novel hydantoin drug hapten analogues are disclosed in copending EP-A-517 325.

Several advantages are realized by use of the above hydantoin derivatives. First, it was found that the active esters of these hydantoin derivatives having short linking chains between the hydantoin nucleus and the active ester group were sufficiently reactive with HRP to prepare an acceptable enzyme label for use with some immobilized antibodies. Derivatives with longer linker groups ($R^2$ plus the bracketed groups) of 8 to 20 atoms between the active ester group and the hydantoin nucleus gave labels that could be bound by all immobilized antibodies tested. Linking chains in which each Z is -NR- which with the adjacent carbonyl forms an amide group, are particularly useful in that hydantoin derivatives containing such chains are more resistant to hydrolysis than chains wherein Z is -O- or -S- so that with the adjacent carbonyl it forms an ester group.

Preparatory Examples

The hydantoin analogues can be made according to the following preparations in which phenytoin analogues, a subclass of hydantoin compounds, are made.

1. Preparation of HD 1, 5,5-Diphenyl-3-{4-[2-(3-succinimidoxycarbonylpropionyloxy)ethylaminocarbonyl]butyl} hydantoin.

Step 1: preparation of 5,5-Diphenyl-3-[4-(2-hydroxyethylaminocarbonyl)butyl]hydantoin.

Part A: First the Acid Chloride is prepared.

A mixture of 3-(4-carboxybutyl)-5,5-diphenyl-2,4-imidazolidinedione (3.52 g, 0.01 mole) thionyl chloride (20 mL), N,N-dimethylformamide (2 drops) and chloroform (50 mL) was stirred at room temperature for 3 hours. The solvent was removed on a rotary evaporator in vacuo, and this product was used directly in the next Part B.

Part B: The Acid Chloride is reacted with Ethanolamine.

The acid chloride in chloroform (50 mL) was added dropwise over 15 minutes to a mixture of ethanolamine (1.2 g, 0.02 mole) and triethylamine (2.4 g, 0.024 mole) in chloroform (100 mL). The mixture was then heated to 60°C for 2 hours and stirred to room temperature for 1 hour. The solution was then washed with 5% hydrochloric acid (2 x 100 mL), washed with saturated sodium bicarbonate solution (100 mL), dried over anhydrous magnesium sulfate, filtered, and the solvent removed on a rotary evaporator. The filtrate was then chromatographed using an aluminum oxide column to give material (3.0 g) showing one spot on TLC. This material was used directly in the next preparation.

Step 2: preparation of 3-{4-[2-(3-Carboxypropionyloxy)ethylaminocarbonyl]-butyl}-5,5-diphenylhydantoin.

The hydroxy compound of Step 1 (3.0 g, 0.0075 mole), succinic anhydride (1.0 g, 0.01 mole), and dimethylaminopyridine (0.9 g, 0.0075 mole) in chloroform (100 mL) were heated at 50-60°C for 4 hours and allowed to cool to room temperature over the weekend.

Dichloromethane (300 mL) was added, and the mixture was washed with 5% hydrochloric acid solution (3 x 100 mL), washed with saturated sodium chloride solution (100 mL), dried over anhydrous magnesium sulfate, filtered, and the solvent removed to give a material that gave one spot on TLC.

Step 3: preparation of HD 1: 5,5-Diphenyl-3-{4-[2-(3-succinimidoxycarbonylpropionyloxy)ethylaminocarbonyl]butyl} hydantoin.

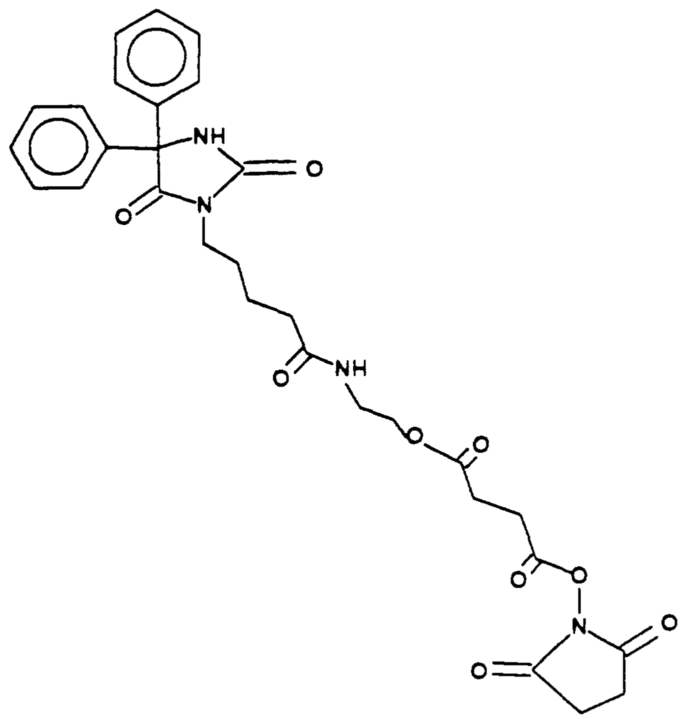

A mixture of the acid from Step 2 (3.0 g, 0.006 mole), N,N'-dicyclohexylcarbodiimide (1.5 g, 0.007 mole), and N-hydroxysuccinimide (0.7 g, 0.006 mole) in chloroform (80 mL) was stirred at room temperature for 20 hours. The mixture was filtered, and the filtrate was concentrated on a rotary evaporator in vacuo. The residue was then chromatographed using silica to give 1.3 g (40% yield).
Anal. calc. for $C_{30}H_{32}N_4O_9$: C, 60.8; H, 5.44; N, 9.45.
Found: C 59.6; H, 5.51, N, 8.91

2. Preparation of HD 2: 5,5-Diphenyl-3-{4-[4-(3-succinimidoxycarbonylpropionyl)-1-piperazinylcarbonyl]butyl}-2,4-imidazolidinedione

Step 1: preparation of 5,5-Diphenyl-3-(1-piperazinylcarbonylbutyl)hydantoin.

Part A: First, 3-[4-(4-Benzyloxycarbonylpiperazinylcarbonyl)butyl]-5,5-diphenyl-2,4-imidazolidinedione was prepared.

The acid chloride prepared as described in the preparation of HD 1, *supra,* Part A (.01 mole) was added dropwise over 15 minutes to a mixture of benzyl 1-piperazinecarboxylate (2.4 g, 0.011 mole) and triethylamine (2.0 g, 0.02 mole) in chloroform (50 mL). This mixture was stirred at room temperature overnight, and dichloromethane (300 mL) was added. The mixture was washed with 5% hydrochloric acid (2 x 100 mL), washed with dilute sodium carbonate solution (100 mL), washed with saturated sodium chloride solution (100 mL), dried over anhydrous magnesium sulfate solution, filtered, and the solvent removed on a rotary evaporator in vacuo. The filtrate was then chromatographed to give an oil, 4.3 g (78% yield) which was used directly in the next step.

Part B: The protected amine of Part A (4.8 g, 0.008 mole) and 30-35% hydrogen bromide acetic acid solution (25 mL) was allowed to stir at room temperature for 1.5 hours. This mixture was then poured into diethyl ether (1 L), and the oil which separated was triturated with fresh portions of ether (3 x 1 L). The oil was dissolved in 10% aqueous sodium hydroxide solution (pH=14) and the aqueous solution extracted with dichloromethane (4 x 100 mL). The combined organic solution was washed with saturated sodium chloride solution (150 mL), dried over

anhydrous magnesium sulfate, filtered, and the solvent removed in a rotary evaporator in vacuo. The filtrate solidifies to give a white solid (2.6 g, 77% yield). This material was used directly in the next step.

Step 2: preparation of 3-{4-[4-(3-Carboxypropionyl)-1-piperazinylcarbonyl]butyl}-5,5-diphenyl-2,4-imidazolidinedione.

A mixture of the amine from step 1 above (2.1 g, 0.005 mole) and succinic anhydride (0.54 g, 0.0054 mole) in chloroform (15 mL) was heated for 30 minutes at 50-60°C and allowed to stand at ambient temperature for 20 hours. Dichloromethane (150 mL) was added, and the mixture was washed with 5% hydrochloric acid (2 x 100 mL), saturated sodium chloride solution (100 mL), dried over anhydrous magnesium sulfate, filtered, and the solvent removed on a rotary evaporator in vacuo to give a white solid, 2.5 g (95%) which was used directly in the next step.

Step 3: preparation of HD 2: 5,5-Diphenyl-3-{4-[4-(3-succinimidoxycarbonylpropionyl)-1-piperazinylcarbonyl]butyl}-2.4-imidazolidinedione.

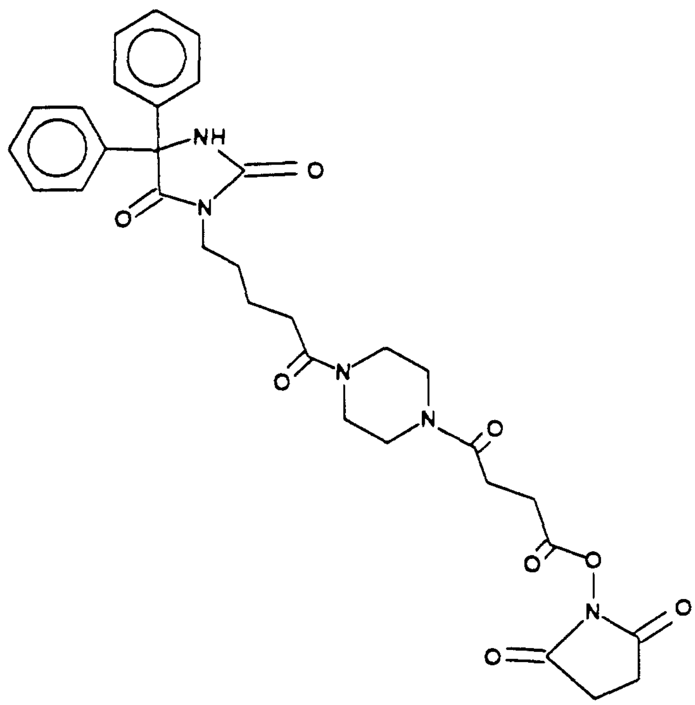

A mixture of the acid from step 2 (1.56 g, 0.003 mole), N,N'-dicyclohexylcarbodiimide (0.64 g, 0.003 mole), and N-hydroxysuccinimide (0.36 g, 0.003 mole) in chloroform (40 mL) was stirred at room temperature over the weekend. The mixture was filtered and the solvent removed from the filtrate on a rotary evaporator in vacuo to give 1.9 g (100% yield). The solid was chromatographed, and the product fraction was dissolved in dichloromethane (200 mL), washed with dilute sodium carbonate solution (2 x 100 mL), dried over anhydrous magnesium sulfate, filtered, and the solvent removed on a rotary evaporator to give a white solid which gives one spot on TLC. Anal. calc. for $C_{32}H_{35}N_5O_8$: C, 62,23; H, 5.71; N, 11.34. Found: C, 59.07; H, 5.40; N, 10.45.

3. Preparation of HD 3, 5,5-Diphenyl-3-{4-[6-(3-succinimidoxycarbonylpropionamido)hexylaminocarbonyl]butyl}-2,4-imidazolidinedione.

Step 1: preparation of 3-[4-(6-Aminohexylaminocarbonyl)butyl]-5,5-diphenyl-2,4-imidazolidinedione.

Part A: preparation of 3-[4-(6-Benzyloxycarbonylaminohexylaminocarbonyl)butyl ]-5,5-diphenyl-2,4-imidazolidinedione.

The acid chloride prepared as an intermediate in the preparation of HD 1 was treated with N-benzyloxycarbonyl-1,6-hexanediamine by the procedures described in step 1 in the preparation of HD 2, to give 7.5 g, 85% yield, of the protected amine.

Part B: The protected amine of Part A was treated with hydrobromic acid-acetic acid by the procedures of Step 1, Part B in the preparation of HD 2, to give the free amine which was used in step 3 without purification.

Step 3: preparation of 3-{4-[6-(3-Carboxypropionamido)hexylaminocarbonyl]butyl)-5,5-diphenyl-2,4-imidazolidinedione.

This compound was prepared using the same procedures as step 2 of the HD 2 preparation. Anal. Calc. for $C_{30}H_{38}N_4O_6$: C, 65.44; H. 6.96; N, 10.17. Found: C, 63.26, H, 7.01; N, 9.39.

Step 4: preparation of HD 3: 5,5-Diphenyl-3-{4- [6- (3-succinimidoxycarbonylpropionamido)hexylaminocarbonyl]butyl)-2,4-imidazolidinedione.

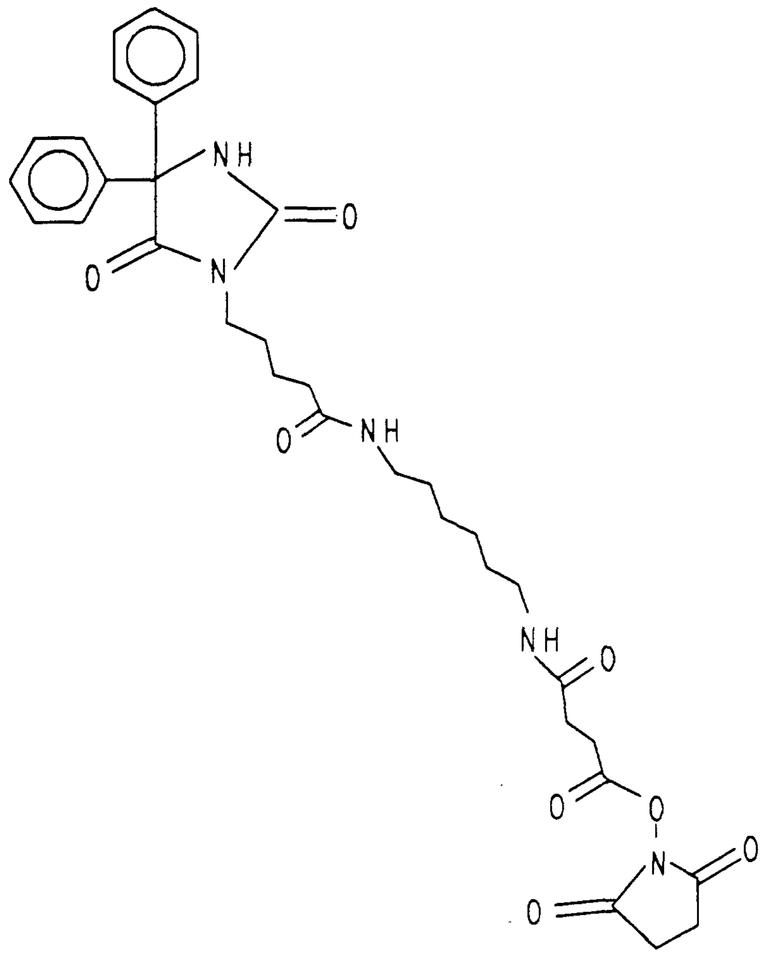

This material was prepared using the procedures of step 3 in the preparation of HD 2 to give 2.6 g (80% yield), mpt 133-134°C. Anal. Calc. for $C_{34}H_{41}N_5O_8$: C, 63.05; H, 6.38; N. 10.81. Found: C, 62.91; H, 6.41; N, 10.69.

Barbiturate Drug Analogues

The following preparatory examples 4 to 8 illustrate the preparation of the barbiturate drug hapten analogues for phenobarbital. The analogues are generally prepared by (1) condensing a barbiturate derivative such, as phenobarbital, with an omega-haloalkanecarboxylate ester, (2) saponifying the ester to the corresponding acid, (3) conversion of the acid to the corresponding acid chloride and (4) condensation of the acid chloride with N-hydroxysuccinimide, or

to further lengthen the linking chain, with a diamine, diol, or aminoalcohol having one of the amine or hydroxy groups blocked, (5) deblocking, condensation with a dicarboxylic acid such as succinic acid, and then condensation with the N-hydroxysuccinimide to produce the analogue.

If desired, the condensation with a half-blocked diamine, diol, or aminoalcohol, and then another diacid can be repeated once or twice more to further lengthen the linking chain. However, the same can be accomplished with fewer steps by using longer chained diacids, diols, diamines, amino alcohols, or haloalkanecarboxylate esters.

4. Preparation of PB 1: 5-Ethyl-5-phenyl-l-{4-[4-(3-Succinimidoxycarbonylpropionyl)-1-piperazinylcarbonyl]butyl}-2,4,6 (1H,3H,5H)pyrimidinetrione

Step 1: Preparation of 5-Ethyl-6-hydroxy-3-(4-methoxycarbonylbutyl)-5-phenyl-2,4(3H,5H)-pyrimidinedione

A mixture of phenobarbital (46.5 g, 0.2 mole) and tetrabutylammonium hydroxide (500 mL, 0.2 mole of 0.4M in water) in dichloromethane (500mL) was prepared and to it was added methyl 5-bromovalerate (39.0 g, o.2 mole). The reaction mixture was stirred vigorously overnight (20 hrs). To this mixture was added saturated sodium chloride solution (100 mL), the organic layer was separated, and the aqueous solution was washed with dichloromethane (2 x 100 mL). The combined organic solution was washed with saturated sodium chloride solution (100mL), dried over anhydrous $MgSO_4$, filtered, and the solvent removed.

Step 2: Preparation of 3-(4-Carboxybutyl)-5-ethyl-6-hydroxy-5-phenyl-2,4(3H,5H)-pyrimidinedione

The 5-ethyl-6-hydroxy-3-(4-methoxycarbonylbutyl)-5-phenyl-2,4(3H,5H)-pyrimidinedione ester (54.0 g, 0.156 mole) of step 1 in dioxane (500 mL), concentrated hydrochloric acid (55 mL), and water (55 mL) was heated at reflux for 4 hrs and at room temperature overnight. The dioxane was removed under reduced pressure, and saturated sodium chloride solution (250 mL) and dichloromethane (400 mL) were added to the residue. The organic layer was separated, and the aqueous solution was extracted with dichloromethane (3 x 150 mL). The combined organic solutions were washed with saturated sodium chloride solution (200 mL), dried over anhydrous $MgSO_4$, filtered, and the solvent removed. To the residue was added diethyl ether, and the mixture was placed in a freezer at -16 °C over the weekend, and then filtered.

Step 3: Preparation of 1-(4-chlorocarbonylbutyl)-5-ethyl-5-phenyl-2,4,6(1H,3H,5H)pyrimidinetrione

A mixture of the acid (6.6 g, 0.2 mole) from preparation 2, thionyl chloride (50 mL), N,N-dimethylformamide (2 drops), and chloroform (80 mL) was stirred at room temperature for 1.5 hrs. The solvent was removed on a rotary evaporator in vacuo, and this product was used directly in the next step 4.

Step 4: Preparation of 1-[4-(4-Benzyloxycarbonyl-1-piperazinylcarbonyl)butyl]-5-ethyl-5-phenyl-2,4,6(1H,3H,5H)-pyrimidinetrione

The acid chloride of step 3 (0.2 mole) in chloroform (75 mL) was added dropwise over 15 minutes to a mixture of benzyl 1-piperazinecarboxylate (6.0 g, 0.030 mole) and triethylamine (4.0 g, 0.04 mole) in chloroform (100 mL). This mixture was stirred at room temperature for 20 hrs, and dichloromethane was then added (300 mL). The mixture was washed with 10% hydrochloric acid solution (3 x 100 mL), washed with saturated sodium chloride solution (100 mL), dried over anhydrous magnesium sulfate, filtered, and the solvent removed. The residue was then chromatographed on $SiO_2$ to give a solid.

Step 5: Preparation of 5-Ethyl-5-phenyl-1-[4-(1-piperazinylcarbonyl)butyl]-2,4,6(1H,3H,5H)pyrimidinetrione Hydrobromide

The protected amine from preparation 4 (6.5 g, 0.012 mole) and 30-35% hydrogen bromide-acetic acid solution (30 mL) was allowed to stir at room temperature for 1.5 hrs. The mixture was then poured into ethyl acetate (2 L), stirred for 1 hr, filtered, and the solid washed with 500 mL ethyl acetate.

Step 6: Preparation of 1-{4-[4-(3-Carboxypropionyl)-1-piperazinylcarbonyl]butyl}-5-ethyl-5phenyl-2,4,6(1H,3H,5H)-pyrimidinetrione

The amine of step 5 (4.8 g, 0.01 mole), succinic anhydride (1.2 g, 0.012 mole), and triethylamine (2.2 g, 0.02 mole) in chloroform (150 mL) were heated for 30 min at 50-60 °C (hot water) and allowed to stir at ambient temperature

for 16 hrs. Dichloromethane (200 mL) was added, the mixture was washed with 10% hydrochloric acid solution (3 x 100 mL), saturated sodium chloride solution (100 mL), dried over anhydrous magnesium sulfate, filtered, and the solvent removed on a rotary evaporator in vacuo to give a white solid, 3.3 g (66%). This material was chromatographed using a $SiO_2$ column to give a white solid.

Step 7: Preparation of 5-Ethyl-5-phenyl-1-(4-[4-(3-Succinimidoxycarbonylpropionyl)-1-piperazinylcarbonyl]butyl}-2,4,6 (1H,3H,5H)pyrimidine-trione

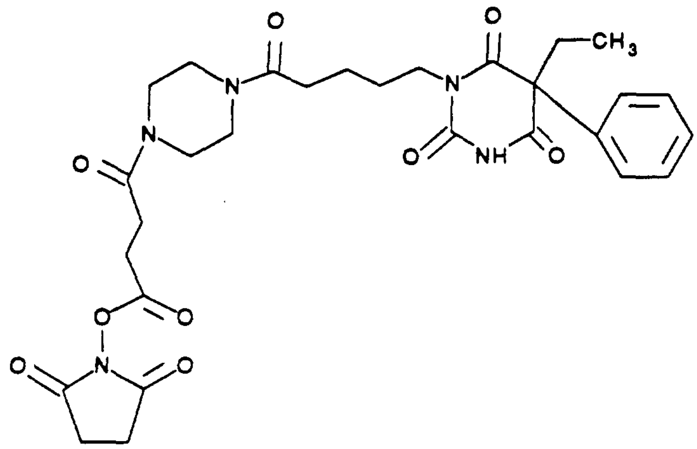

A mixture of the acid from step 6 (3.4 g, 0.007 mole), N,N'-dicyclohexylcarbodiimide (1.6 g, 0.008 mole), and N-hydroxysuccinimide (1.0 g, 0.008 mole) in chloroform (75 mL) was stirred at room temperature for 20 hrs. The mixture was filtered, and ethyl acetate (100 mL) was added. The organic solution was washed with water (2 x 100 mL), saturated sodium chloride solution (50 mL), dried over anhydrous magnesium sulfate, filtered, and the solvent removed on a rotary evaporator under vacuo. A portion of the solid was chromatographed to give a white solid.

5. Preparation of PB 3, 5-Ethyl-5-phenyl-1-{4-[2-(3-succinimidoxycarbonylpropionyloxy)ethylaminocarbonyl]butyl}-2,4,6(1H,3H,5H)-pyrimidinetrione

Step 1: Preparation of 5-Ethyl-1-[4-(2-hydroxyethylaminocarbonyl)butyl]-5-phenyl-2,4,6(1H,3H,5H)pyrimidinetrione

This material was prepared as outlined in step 4 of preparatory example 4 except using 2-hydroxyethylamine in place of the benzyl 1-piperazinecarboxylate.

Step 2: Preparation of 1-{4-[2-(3-Carboxypropionyloxy)ethylaminocarbon yl]butyl}-5-ethyl-5-phenyl-2,4,6(1H,3H,5H)-pyrimidinetrione

A mixture of the product from step 1 (2.9 g, 0.007 mole), succinic anhydride (0.7 g, 0.007 mole), and dimethylaminopyridine (0.9 g, 0.007 mole) in chloroform (100 mL) was heated with hot water (50-60 °C) for 30 min and then stirred at room temperature for 3 days. Dichloromethane (300 mL) was added, and the mixture was washed with 10% hydrochloric acid solution (2 x 100 mL), washed with saturated sodium chloride solution (100 mL), dried over anhydrous magnesium sulfate, filtered, and solvent removed to give an oil which was used directly in the next step.

Step 3: Preparation of PB 3, 5-Ethyl-5-phenyl-1-{4-[2-(3-succinimidoxycarbonylpropionyloxy)ethylaminocarbonyl] butyl}-2,4,6(1H,3H,5H)-pyrimidine-trione

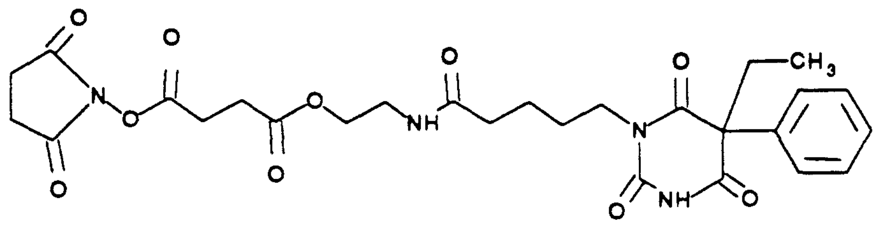

This material was prepared using the procedure outlined in step 7 of preparatory example 4 starting with the acid of step 2 of this example.

6. Preparation of PB 4, 5-Ethyl-5-phenyl-1-{4-[3-(3-succinimidoxycarbonylpropionamido)propylaminocarbonyl]butyl)-2,4,6(1H,3H,5H)-pyrimidinetrione

Step 1: Preparation of 1-[4-(3-Benzyloxycarbonylaminopropylaminocarbonyl)butyl]-5-ethyl-5-phenyl-2,4,6(1H,3H,5H)-pyrimidinetrione

This material was prepared using the procedure outlined in step 4, preparatory example 4, except substituting N-benzyloxycarbonyl-1,3-propanediamine for the benzyl 1-piperazinecarboxylate, and the crude material was used in the next step.

Step 2: Preparation of 1-[4-(3-Aminopropylaminocarbonyl)butyl]-5-ethyl-5-phenyl-2,4,6(1H,3H,5H)-pyrimidinetrione Hydrobromide

This material was prepared as in step 5, preparatory example 4 (except starting with the amide of step 1 of this example to give an oil when poured into ethyl ether.

Step 3: Preparation of 1-{4-[3-(3-Carboxypropionamido)propylaminocarbonyl]-butyl}-5-ethyl-5-phenyl-2,4,6(1H,3H, 5H)-pyrimidinetrione

This material was prepared by the procedure of step 6, preparatory example 4, except starting with the amine from step 2 of this example to give the acid.

Step 4: Preparation of PB 4, 5-Ethyl-5-phenyl-1-{4-[3-(3-succinimidoxycarbonylpropionamido)propylaminocarbonyl] butyl}-2,4,6(1H,3H,5H)-pyrimidinetrione

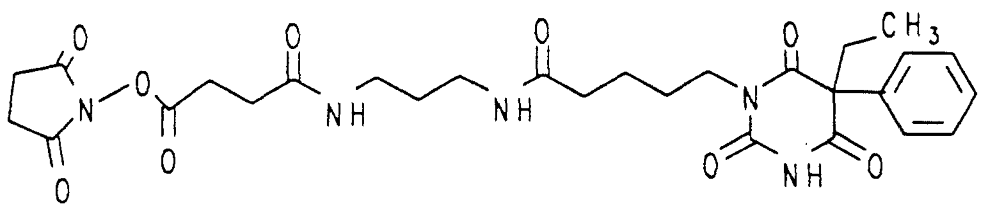

This material was prepared using the procedure of step 7, preparatory example 4 except starting with the acid of step 3 of this example.

7. Preparation of PB 5, 5-Ethyl-5-phenyl-1-{4-[6-(3-succinimidoxycarbonylpropionamido)-hexylaminocarbonyl]butyl}-2,4,6(1H,3H,5H)-pyrimidinetrione

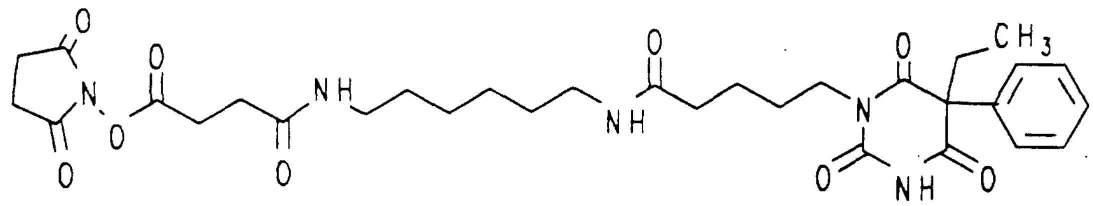

This compound was prepared by the sequence of reactions of preparation example 6 except substituting N-benzyloxycarbonyl-1,6-hexanediamine for the benzyloxycarbonyl-1,3 propanediamine in step 1, and the respective reaction products thereafter in steps 2, 3, and 4 of preparation example 6.

Labeled Drug Hapten Analogues

We have prepared new labeled drug hapten analogues of the above prepared barbiturate and hydantoin analogues which are useful in competitive immunoassays for barbiturates and hydantoin drugs, particularly phenobarbital and phenytoin. The labels are those commonly used in immunoassays having an amine or sulfhydryl group commonly used with analytes or analyte analogs in competitive immunoassays such as enzymes, visible dyes, leuco dyes, fluorescent dyes, radioactive materials, and so forth.

Useful labels are enzymes such as alkaline phosphatase (ALP), glucose oxidase (GOD) and horseradish peroxidase (HRP) or amine-enriched horseradish peroxidase (AHRP).

The labeled drug hapten analogues are prepared with a new process comprising the steps of:

1) contacting a label having a nucleophilic group thereon such as an amine or sulfhydryl group, with an excess of a barbiturate or hydantoin drug hapten analogue described *supra.* Preferably the analogues and the label are dissolved in a water miscible organic solvent such as N,N-dimethylformamide, dimethyl sulfoxide (DMSO) or mixture of solvent and water (buffered) before mixing together; and
2) removing the unused active ester and condensation by-products, preferably by dialysis.

The examples provided hereinafter illustrate the preparation of the new labeled analogues of this invention. The labeled analogues were prepared using phenytoin and phenobarbital drug hapten analogues.

Labeled Hydantoin Analogues

Example 1 - Preparation of an Amine Enriched HRP labeled hydantoin HD 1 (containing an extended linker, label AHRP-HD 1, Label A).

HD 1 was dissolved in 1.452 mL dry DMF containing 10 mM 4'-hydroxyacetanilide (DMF 4'-HA).

Amine-enriched HRP was prepared as follows. Dry HRP was dissolved in 0.1 M MES buffer, pH 5.5, to achieve a final concentration of 2.5 x $10^{-6}$ mol (100 mg) in 10 mL of buffer (MES = 2-(N-morpholino)-ethanesulfonic acid). The protein concentration was determined by A403 measurement using the conversion factor A403 1 mg/mL = 2.24. The HRP solution was combined with 1.5 x 10-3 mol (275 mg) of L-lysine monohydrochloride dissolved in 10 mL of 0.1 M MES buffer at pH 5.5. A solution of freshly prepared 1-ethyl-3-(3-dimethylaminopropyl)carbodiimide hydrochloride (EDC, 5 x 10-4 mol, 960 mL) in MES buffer was added. The container was capped and mixed overnight at room temperature. The reaction was dialyzed against 0.02 M MOPS buffer at pH 7.0 (3 L at 10°C). The dialysis buffer was changed 3X. MOPS = 3-(N-morpholino)propanesulfonic acid.

Prior to reaction, a sample of the amine-enriched HRP was exchanged from MOPS buffer into 0.1 M EPPS buffer, pH 8.0, using 30,000 NMWL (nominal molecular weight limit cutoff) Centricells centrifugal ultrafilters. This sample was then diluted to obtain a solution with a final concentration of 10 mg/mL.

The amine-enriched HRP (AHRP) (1 mL) was combined with 500 µL of a 10 mM solution of 4'-hydroxyacetanilide in dimethylformamide (DMF 4'-HA) with vortex mixing and was then placed in a 42°C water bath. An HD 1 solution prepared by dissolving 21 mg of HD 1 in 1.452 mL of dry DMF 4'-HA solution (500 µL) was added dropwise to the AHRP with vortex mixing so that the molar ratio of phenytoin/HRP was 50/1. The reaction was incubated for 1 hour in a 42°C water bath with gentle shaking.

The reaction was placed in Spectrapor #2 dialysis tubing along with an additional 0.5 mL of DMF 4'-HA/0.1 M EPPS (1:1) used to rinse the reaction container.

The reaction mixture was dialyzed as follows:

a) 1 L DMF 4'-HA/0.1 M EPPS. pH 8.0, (1:1) at 42°C for 1 hr;
b) Dialysis condition a) was repeated 1 time;
c) 2 L 0.1 M EPPS, pH 8.0, containing 0.1% BSA at 8°C for 15 hrs
d) 2 L of 0.1 M EPPS, pH 8.0, at 8°C for 3 hours;
e) 3 L of 0.04 M tris(hydroxymethyl)-aminomethane hydrochloride (Tris HCl)/0.15 M NaCl, PH 7.5, at 8°C for 3 hours; and
f) Dialysis condition e) was repeated 1 time for 3 hrs;

Following dialysis, 0.02% merthiolate was added as a preservative, and the AHRP-HD 1 was stored refrigerated.

Example 2 - Preparation of an Amine Enriched HRP labeled HD 2 (containing an extended linker with an amide bond, label AHRP-HD 2, Label B).

HD 2 (15.5 mg) was dissolved in 1.031 mL dry DMF containing 10 mM 4'-hydroxyacetanilide (DMF 4'-HA).

A solution of AHRP (10 mg/mL, 1 mL in 0.1 M EPPS, pH 8.0), prepared as described in label Preparatory example 1, was combined with 500 µL of DMF 4'-HA with vortex mixing and was then placed in a 42°C water bath. The HD 2 solution described above (500 µL) was added dropwise to the AHRP with vortex mixing so that the molar ratio was 50/1. The reaction was incubated for 1 hour in a 42°C water bath with gentle shaking.

The reaction product was placed in Spectrapor #2 dialysis tubing and dialyzed as follows:

a) 1 L DMF 4'-HA/O.1 M EPPS, pH 8.0 (1:1) at 42°C for 1 hr;
b) Dialysis condition a) was repeated 1 time;
c) 1.5 L 0.1 M EPPS, pH 8.0, containing 0.1% bovine serum albumin (BSA) at 8°C for 1.5 hr;
d) 1.5 L 0.1 M EPPS, pH 8.0, at 8°C for 18 hrs;
e) 1.5 L 0.04 M Tris HCl/0.15M NaCl, pH 7.5, at 8°C for 2 hrs; and
f) Dialysis condition e) was repeated 1 time for 4 hrs.

Following dialysis, 0.02% merthiolate was added as a preservative. The labeled hydantoin derivative was stored in a refrigerator.

Example 3 - Preparation of AHRP-HD 3 (containing an extended linker with an amide bond label AHRP-HD 3, Label C).

HD 3 (9.2 mg) was dissolved in 1 mL dry DMF containing 10 mM 4'-hydroxyacetanilide (DMF 4'-HA).

A solution of AHRP, prepared as described in label preparatory example 1, was dialyzed into 0.1 M EPPS buffer, pH 8.0. The final concentration was determined to be 5.71 mg/ml.

The AHRP (1 mL) was combined with 500 µL of DMF 4'-HA with vortex mixing and was then placed in a 42°C water bath. The HD 3 solution described above (500 µL) was added to the AHRP dropwise with vortex mixing so that the molar ratio of HD 3/AHRP was 50/1. The reaction was incubated for 1 hour in a 42°C water bath with gentle shaking. The reaction was placed in Spectrapor #2 dialysis tubing along with an additional 0.5 mL of DMF 4'-HA/0.1 M EPPS (1:1) used to rinse the reaction container.

The reaction was dialyzed as follows:

a) 1 L DMF 4'-HA/0.1 M EPPS, pH 8.0, (1:1) at 42°C for 1 hour;
b) dialysis condition a) was repeated 1 time;
c) 1.5 L 0.1 M EPPS, pH 8.0, containing 0.1% BSA at 5°C for 15 hours;
d) 1.5 L 0.1 M EPPS, pH 8.0, for 8 hours;
e) 2 L 0.02 M 3-morpholinopropanesulfonic acid (MOPS), pH 7.0. at 5°C for 13 hours; and
f) Dialysis condition e) was repeated 2 times.

Following dialysis, merthiolate was added to a concentration of 0.02% as a preservative, and the label was stored refrigerated.

Labeled Barbiturate Drug Hapten Analogues

The following examples demonstrate the preparation of labeled barbiturate drug hapten analogues.

Example 4 - Preparation of Amine Enriched HRP labeled PB 2; (label AHRP-PB 2. Label D)

PB 2 was dissolved in DMSO to yield a 10.7 mg/mL solution (1.25 x $10^{-2}$ M). Then 500 µL of this solution was added to amine enriched HRP/DMSO solution (prepared similar to AHRP/DMF) dropwise while vortex mixing. The molar ratio of the phenobarbital/HRP was 50/1.

Incubation was performed at room temperature for 4 hours with shaking at 2400 rpm. The sample was transferred to Spectrapor #2 dialysis tubing along with an additional 1 mL of dialysate to rinse the reaction container. The label was dialyzed into 0.02 M MOPS buffer, pH 7.0, at 5-10°C. This dialysis condition was repeated three times with 2-3 L of buffer each time. Following dialysis, 0.02% merthiolate was added as a preservative, and the label was stored refrigerated.

Example 5 - Preparation of Amine Enriched HRP-PB 3; (AHRP-PB3 Label E containing an Extended Linker)

Amine-enriched HRP was exchanged from MOPS buffer into 0.1 M EPPS buffer, pH 8.0, using a Centricell centrifugal ultrafilter (30,000 nominal molecular weight limit). This sample was then diluted to 4.6 mL, 0.743 mg/ml.

One mL of the HRP was added to a small vial (1.85 x 10-5 M). 500 µL of dimethylformamide, Aldrich 22,705-6, containing 10 mM 4'-hydroxyacetanilide (DMF 4'-HA) was added to the vial, vortexed, and placed in a 42°C water bath.

Meanwhile, PB-3 was dissolved in DMF 4'-HA to yield a 2.12 mg/ml solution (3.70 x $10^{-3}$ M). 500 µL of this solution was added to the HRP/DMF 4'-HA solution dropwise while vortex mixing. The molar ratio of the phenobarbital/HRP was 100/1.

Incubation was performed at 42°C for 1 hour with gentle shaking in a water bath. The sample was transferred to Spectrapor #2 dialysis tubing along with an additional 1 mL of DMF 4'-HA/0.1 M EPPS (1:1) used to rinse the reaction container.

The reaction was dialyzed as follows:

a) 1 L DMF 4'-HA/0.1 M EPPS, pH 8.0 (1:1) at 42°C for 1 hr;
b) Dialysis condition a) was repeated once;
c) 1.5 L 0.1 M EPPS, pH 8.0, containing 0.1% bovine serum albumin (BSA) at 5°C overnight;
d) 1.5 L 0.1 M EPPS, PH 8.0, at 5°C, 8 hrs;
e) 2.0 L 0.02 M MOPS, pH 7.0 at 5°C, for at least 8 hrs; and
f) Dialysis condition e) was repeated twice.

Following dialysis, 0.02% merthiolate was added as a preservative, and the label was stored refrigerated.

Example 6 - Preparation of an Amine Enriched HRP-PB 1; (Label AHRP-PB 1, Label F, an Active Ester of a Phenobarbital Hapten analogue Containing an Extended Linker with an Amide Bond)

Amine-enriched HRP was prepared and exchanged into 0.1 M EPPS buffer, pH 8.0, to yield a, 10 mg/mL solution (2.5 x 10-4 M). Label F was made using 5 mL (50 mg) amine-enriched HRP. 2.5 mL DMSO was added slowly, while stirring over a magnetic stir plate. The solution was stirred for 15 minutes at room temperature.

Meanwhile, PB 1 was dissolved in DMSO to yield a 14.9 mg/ml solution. 2.5 mL of this solution was added to the HRP/DMSO solution slowly while stirring. The molar ratio of the phenobarbital/HRP was 50/1.

Incubation was performed at room temperature for 5 hours with shaking at 2400 rpm. The sample was transferred to Spectropor #2 dialysis tubing along with additional dialysate to rinse the reaction container. The label was dialyzed into 0.02 M MOPS buffer, pH 7.0, at 5-10°C. This dialysis condition was repeated three times with 3 L of buffer each time. Following dialysis, 0.02% merthiolate was added as a preservative, and the labels were stored refrigerated.
Immunocompetence

The following tests demonstrate the immunocompetence of the labels 1-6, supra.

Example 7 - Immunocompetency of label AHRP-HD 1 (Label A).

In this example, the ability of several immobilized antibodies ($DilAs_8$, $DilAs_9$, $DilAs_{14}$, $DilAs_{16}$, and $DilAs_{21}$) to bind AHRP-HD 1 (label A) from label preparatory example 1) is examined.

(a) Polymer bead samples, each sample having one of the above-identified types of antibodies covalently bound thereto were prepared using methods and materials as described in EP-A-323 692.

(b) The ability of the immobilized antibodies to bind AHRP-HD 1 (label A) was determined as follows:

Each of the various antibody beads were serially diluted with PBS containing 1% BSA to give concentrations between 500 and 0.50 nM antibody binding sites. The bead dilutions were mixed with equal volumes of the label at 10 x $10^{-11}$ M. Following a 1 hour incubation, the beads were pelleted by centrifugation. A sample (100 μL) of the supernatant was mixed with 100 μL of substrate (o-phenylenediamine/$H_2O_2$). The rates of color development at 450 nm were compared with those of standards to calculate the amount of phenytoin-HRP label remaining in solution. The amount of label bound to immobilized antibody at the highest antibody concentration tested (250 nM binding sites) is reported.

| % Label Bound at 250 nM | Antibody Binding Sites Label A (Extended Linker) |
|---|---|
| $DilAs_8$ | 93 |
| $DilAs_9$ | 94 |
| $DilAs_{14}$ | 90 |
| $DilAs_{16}$ | 96 |
| $DilAs_{21}$ | 97 |

These results show that these antibodies recognize very well AHRP-HD 1 Labels (label A).

Example 8 - Hydrolytic stability of AHRP-HD 2 (label B)

This example illustrates the hydrolytic stability of a labeled phenytoin derivative of the invention having an amide bond in the linking chain between the label and the phenytoin nucleus AHRP-HD 2, (label B).

Beads having immobilized Kallestad antibodies were prepared as described in EP-A-323 692.

AHRP-HD 2 (label B) was diluted to 1 x $10^{-10}$ M in PBS containing 1% BSA adjusted to pH 7.3 or 8.5. The label was incubated at room temperature for 6 days. The label was tested for binding by immobilized antibody after 2 days and 6 days as follows:

Kallestad 52-2 antibody beads were serially diluted with PBS containing 1% BSA to give concentrations between 500 and 0.50 nM antibody binding sites. The bead dilutions were mixed with equal volumes of labels at 10 x $10^{-11}$ M. Following a 1 hour incubation, the beads were pelleted by centrifugation. A sample (100 uL) of the supernatant was mixed with 100 uL of substrate (o-phenylenediamine/$H_2O_2$). The rates were compared with those of standards to calculate the amount of label remaining in solution. The amount of label bound to immobilized antibody at the highest antibody concentration tested (250 nM binding sites) is reported.

| Percent Label Bound at 250 nM | Antibody Binding Sites Label B (Amide Bond) | |
|---|---|---|
| | pH 7.3 | pH 8.5 |
| 0 days | 100 | -- |
| 2 days | 98 | 99 |
| 6 days | 99 | 100 |

The results show that binding of AHRP-HD 2 (label B) containing the amide bond in the linking chain showed no degradation over this time period. This indicates that label B will resist degradation due to hydrolysis. Such hydrolysis could cause a shift in the assay response with time.

Example 9 - Comparison of Phenobarbital-HRP Labels Prepared with Valerate and Extended Linkers

In this example, the ability of several immobilized antibodies (Kall 1571 and PbAs9) to bind a label with the valerate linker (label D, AHRP-PB 2) and a label with an extended linker (label F, AHRP-PB 1) were compared.

Immobilized antibody bead samples were prepared as follows:

Polymer beads (30 mg) [poly(styrene-co-p-vinylbenzyl 2-chloroethyl sulfone) (95:5 molar ratio)] were dispersed in 1 mL buffer (0.1 M EPPS, pH 8.5) and 0.3 mg of antibody (Kall 1571 or PbAs9) was added. The total volume was 1.5 mL. The mixture was rotated end over end at room temperature for 4 hours. Then 0.3 mL of a 10% solution of BSA was added, and the supernatants were removed and analyzed for unbound antibody using an anti-mouse IgG. The amount of antibody bound to the surface was calculated using ELISA. The pellets were washed 3 times with PBS, pH 7.2, by resuspending in the buffer and centrifuging. The final redispersion was in 1.8 mL of PBS; merthiolate was added to a concentration of 0.02%, and the preparations were stored at 4°C until use.

The ability of the immobilized antibodies to bind label was determined as follows:

Each of the various antibody beads was serially diluted with PBS containing 1% BSA to give concentrations between 200 and 0.50 nM antibody binding sites. The bead dilutions were mixed with equal volumes of phenobarbital-HRP labels at 10 x 10-11 M. Following a 1-hour incubation, the beads were pelleted by centrifugation. A sample (100 µL) of the supernatant was mixed with 100 µL of substrate (o-phenylenediamine/$H_2O_2$). The rates of color development at 450 nm were compared with those of standards to calculate the amount of phenobarbital-HRP label remaining in solution. The amount of label bound to immobilized antibody at the highest antibody concentration tested (100 nM binding sites) is reported.

| % Label Bound at 100 nM Antibody Binding Sites | | |
|---|---|---|
| | Label D | Label F |
| Kall 1571 | 41% | 76% |
| PbAs9 | 49% | 73% |

These results indicate that these antibodies show improved recognition of labels prepared with haptens having the extended linking group used in labeled drug hapten analogues provided by the invention.

## Claims

**1.** Labeled drug analogues comprising:

(A) an enzyme label;
(B) a drug nucleus selected from a hydantoin or a barbiturate nucleus; and
(C) a linking chain linking the 3-position of the drug nucleus to the label through a carbonyl bridge; wherein the linking chain, excluding the said carbonyl bridge, has 12 to 40 atoms consisting of:

(I) two or more $C_1$ to $C_{10}$ alkylene groups;
optionally in combination with
one or more phenylene groups, and/or
one or more 5 to 7 membered heterocyclic rings joined into the linking group through ring nitrogen atoms;
or
(II) one $C_1$ to $C_{10}$ alkylene group in combination with one or more phenylene groups and one or more 5 to 7 membered heterocyclic rings joined into the linking group through ring nitrogen atoms;

said groups and rings being bonded to each other through chemical groups selected from

(a) esters, (-COZ-), where Z is O or S;
(b) amides, (-CONH-),
(c) hetero atoms selected from -O-, -S-, and -NR-; wherein R represents $C_1$ to $C_6$ alkyl; and
(d) carbonyl.

**2.** The labeled analogue of claim 1 wherein the linking chain includes a heterocyclic group selected from 1,4-piperazinylene, 2-5-dimethyl-1-,4-piperazinylene, 1,3-imidazolidinylene, and 1, 3-hexahydrodiazepinylene groups.

**3.** The labeled analogue of claim 1 conforming to the structure:

(I)

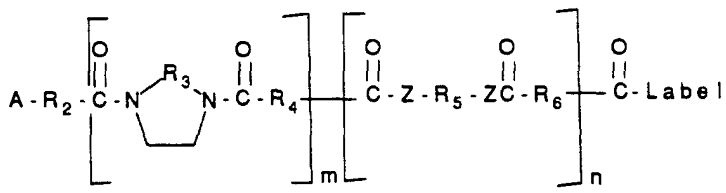

wherein

A represents a hydantoin nucleus of the structure

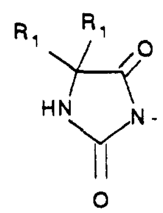

or a barbiturate nucleus of the structure

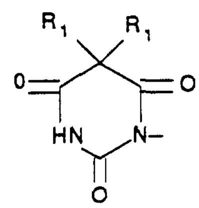

;

$R^1$ each independently represents hydrogen, alkyl of 1 to 10 carbon atoms, unsubstituted or substituted phenyl;
$R^2$, $R^4$, $R^5$, and $R^6$, each independently represents $C_1$ to $C_{10}$ alkylene or such alkylene groups interrupted with at least one or more ester groups, amide groups, -O-, -S-, and -NR-;
$R^3$ represents $C_1$ to $C_3$ alkylene;
Z represents -O-, -S-, or -NR-, wherein R represents hydrogen or $C_1$ to $C_6$ alkyl;
m is 0, 1, or 2;
n is 0, 1, or 2;
m + n > 0; and

the total number of atoms comprised in m, n and $R^2$ is 12 to 40;
LABEL is an enzyme;
and further provided that (i) at least one of the $R^1$ groups is substituted or unsubstituted phenyl; (ii) one of $R^4$, $R^5$, and $R^6$ can be phenylene; and (iii) the bracketed components of structure I can appear therein in any order.

**4.** The labeled analogue of claim 3 wherein:

$R^1$ each independently represents hydrogen, methyl, propyl, hexyl, decyl, unsubstituted phenyl or phenyl substituted with alkyl of 1 to 6 carbon atoms, nitro, halogen, cyano, and alkoxy of 1 to 6 carbon atoms;
$R^2$, $R^4$, $R^5$, and $R^6$, each independently represent alkylene selected from ethylene, trimethylene, butylene, pentylene, octylene or such alkylene interrupted with at least one or more ester groups, amide groups, -O-, -S-, and -NR-;

$R^3$ represents methylene, ethylene, or trimethylene;
Z represents -NR-, wherein R represents hydrogen, methyl, propyl, or hexyl; and
LABEL represents an enzyme.

**5.** The labeled analogue of claim 1 wherein the label is selected from horseradish peroxidase (HRP) or amine enriched horseradish peroxidase (AHRP); the drug nucleus is phenytoin or phenobarbital and the linking group is selected from:

tetramethylenecarbonyliminohexamethyleneiminocarbonylethylenecarbonyl,
tetramethylenecarbonyl-1,4-piperazinylenecarbonylethylenecarbonyl, and
tetramethylenecarbonyliminoethyleneoxycarbonylethylenecarbonyl.

**6.** A method of making the labeled drug analogues of any one of the preceding claims comprising the steps of:

(1) contacting a label having an amine or sulfhydryl group, with an excess of a drug analogue that comprises:

(a) an active ester group,such as succinimidoxycarbonyl;
(b) a drug nucleus selected from a hydantoin nucleus or a barbiturate nucleus;
(c) a linking chain linking the active ester group to the hydantoin or barbiturate nucleus;

wherein the linking chain is as defined in claim 1, and
(2) removing the unused active ester and condensation by-products, preferably by dialysis.

**7.** The method of claim 6 wherein the drug analogue and the label are dissolved in a water-miscible organic solvent or mixture of solvent and water before being contacted together.

**8.** A labeled drug analogue according to claims 1 to 4 wherein the linking chain has 12 to 18 atoms.

**9.** A labeled drug analogue according to claims 3, 4, or 5 wherein Z is -NR- .

**Patentansprüche**

**1.** Markierte Wirkstoff-Analoga, umfassend:

(A) eine Enzym-Markierung;

(B) einen Wirkstoffkern, ausgewählt aus einem Hydantoin- oder einem Barbiturat-Kern; und

(C) eine verbindende Kette, die die 3-Position des Wirkstoffkerns mit der Markierung über eine Carbonylbrücke verbindet; wobei die verbindende Kette, ausgenommen die Carbonylbrücke, 12 bis 40 Atome aufweist, bestehend aus:

(I) zwei oder mehr $C_1$- bis $C_{10}$-Alkylengruppen;
gegebenenfalls in Kombination mit einer oder mehreren Phenylengruppen, und/oder einem oder mehreren 5- bis 7-gliedrigen heterozyklischen
Ringen, die in die verbindende Gruppe über Ring-Stickstoff-Atome eingebunden sind;
oder

(II) eine $C_1$- bis $C_{10}$-Alkylengruppe in Kombination mit einer oder mehreren Phenylengruppen und einem oder mehreren 5- bis 7-gliedrigen heterozyklischen Ringen, die in die verbindende Gruppe über Ring-Stickstoff-Atome eingebunden sind;

wobei die Gruppen und Ringe miteinander über chemischen Gruppen verbunden sind, die ausgewählt sind aus

(a) Estern, (-COZ-), worin Z O oder S ist;

(b) Amiden, (-CONH-),

(c) Heteroatomen, ausgewählt aus -O-, -S-, und -NR-; worin R $C_1$- bis $C_6$-Alkyl repräsentiert; und

(d) Carbonyl.

**2.** Markiertes Analogon nach Anspruch 1, wobei die verbindende Kette eine heterozyklische Gruppe beinhaltet, die aus 1,4-Piperazinylen-, 2,5-Dimethyl-1,4-piperazinylen, 1,3-Imidazolidinylen- und 1,3-Hexahydrodiazepinylen-Gruppen ausgewählt ist.

**3.** Markiertes Analogon nach Anspruch 1, entsprechend der Struktur:

(I)

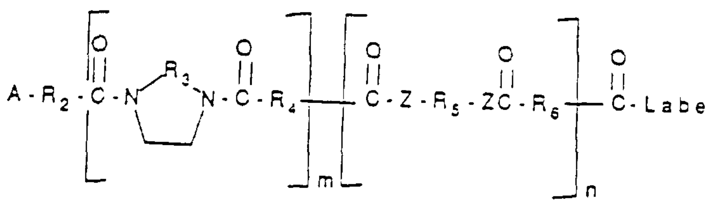

worin

A einen Hydantoin-Kern der Struktur

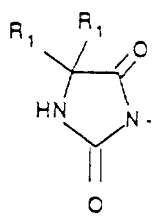

oder einen Barbiturat-Kern der Struktur

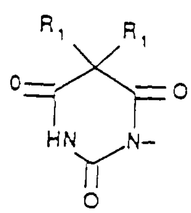

repräsentiert;

worin $R^1$ unabhängig voneinander Wasserstoff, Alkyl aus 1 bis 10 Kohlenstoffatomen, nicht-substuiertes oder substituiertes Phenyl repräsentiert;

$R^2$, $R^4$, $R^5$ und $R^6$ unabhängig voneinander $C_1$- bis $C_{10}$-Alkylen repräsentieren oder solche Alkylengruppen, die mit mindestens einer oder mehreren Estergruppen, Amidgruppen, -O-, -S- und -NR-, unterbrochen sind;

$R^3$ $C_1$- bis $C_3$-Alkylen repräsentiert;

Z -O-, -S-, -NR- repräsentiert, worin R Wasserstoff oder $C_1$- bis $C_6$-Alkyl repräsentiert;

m ist 0, 1 oder 2;

n ist 0, 1 oder 2;

m + n > 0; und

die Gesamtzahl der Atome, die von m, n und $R^2$ umfaßt sind, 12 bis 40 beträgt;

LABEL ist ein Enzym;

und weiter vorausgesetzt, daß (i) mindestens eine der $R^1$-Gruppen substituiertes oder nicht-substituiertes Phenyl ist;

(ii) einer von $R^4$, $R^5$ und $R^6$ Phenylen sein kann; und

(iii) die eingeklammerten Komponenten von Struktur I in jeder Reihenfolge auftreten können.

**4.** Markiertes Analogon nach Anspruch 3, wobei:

$R^1$ unabhängig voneinander Wasserstoff, Methyl, Propyl, Hexyl, Dezyl, nicht-substituiertes Phenyl oder mit Alkyl von 1 bis 6 Kohlenstoffatomen substituiertes Phenyl, Nitro, Halogen, Cyano und Alkoxy von 1 bis 6 Kohlenstoffatomen repräsentiert;

$R^2$, $R^4$, $R^5$ und $R^6$ unabhängig voneinander Alkylen repräsentiert, ausgewählt aus Ethylen, Trimethylen, Butylen, Pentylen, Octylen oder einem solchen Alkylen, das mit mindestens einer oder mehreren Estergruppen, Amidgruppen, -O-, -S- und -NR-, unterbrochen ist;

$R_3$ Methylen, Ethylen oder Trimethylen repräsentiert;

Z -NR- repräsentiert, worin R Wasserstoff, Methyl, Propyl oder Hexyl repräsentiert; und

LABEL ein Enzym repräsentiert.

**5.** Markiertes Analogon nach Anspruch 1, wobei die Markierung aus Meerrettichperoxidase (HRP) oder Amin angereicherter Meerrettichperoxidase (AHRP) ausgewählt ist; der Wirkstoffkern Phenytoin oder Phenobarbital ist und die verbindende Gruppe ausgewählt ist aus:
Tetramethylencarbonyliminohexamethyleniminocarbonylethylencarbonyl, Tetramethylencarbonyl-1,4-Piperazinylencarbonylethylencarbonyl, und Tetramethylencarbonyliminoethylenoxycarbonylethylencarbonyl.

**6.** Verfahren zur Herstellung der markierten Wirkstoff-Analoga nach einem der voranstehenden Ansprüche, umfassend die Schritte aus:

(1) In Kontakt bringen einer Markierung, die eine Amin- oder Sulfhydrylgruppe aufweist, mit einem Überschuß eines Wirkstoff-Analogons, das umfaßt:

(a) eine aktive Estergruppe wie etwa Succinimidoxycarbonyl;

(b) einen Wirkstoffkern, der ausgewählt ist aus einem Hydantoin-Kern oder einem Barbituratkern;

(c) eine verbindende Kette, die die aktive Estergruppe mit dem Hydantoin-oder Barbiturat-Kern verbindet;

wobei die verbindende Kette wie in Anspruch 1 definiert ist; und

(2) Entfernen des unverbrauchten aktiven Esters und der Kondensationsnebenprodukte, bevorzugt durch Dialyse.

**7.** Verfahren nach Anspruch 6, wobei das Wirkstoff-Analogon und die Markierung in einem wassermischbaren organischen Lösungsmittel oder einem Gemisch aus Lösungsmittel und Wasser gelöst werden, bevor sie miteinander

in Kontakt gebracht werden.

**8.** Markiertes Wirkstoff-Analogon nach den Ansprüchen 1 bis 4, wobei die verbindende Kette 12 bis 18 Atome aufweist.

**9.** Markiertes Wirkstoff-Analogon nach den Ansprüchen 3, 4 oder 5, worin Z -NR- ist.

**Revendications**

**1.** Analogues de médicament marqués comprenant :

(A) un marqueur enzymatique;
(B) un noyau de médicament choisi parmi un noyau hydantoïne ou un noyau barbiturate, et
(C) une chaîne de liaison liant la position 3 du noyau de médicament au marqueur par l'intermédiaire d'un pont carbonyle; la chaîne de liaison, à l'exclusion dudit pont carbonyle, possédant 12 à 40 atomes et étant constituée de :

(I) au moins deux groupes alkylène en $C_1$ à $C_{10}$, éventuellement en combinaison avec un ou plusieurs groupes phénylène, et/ou un ou plusieurs noyaux hétérocycliques à 5 - 7 chaînons reliés dans le groupe de liaison par l'intermédiaire d'atomes d'azote du noyau,
ou
(II) un groupe alkylène en $C_1$ à $C_{10}$, en combinaison avec un ou plusieurs groupes phénylène, et un ou plusieurs noyaux hétérocycliques à 5 - 7 chaînons reliés dans le groupe de liaison par l'intermédiaire d'atomes d'azote du noyau;

lesdits groupes et noyaux étant liés les uns aux autres par l'intermédiaire de groupes chimiques choisis parmi

(a) des esters, (-COZ-), où Z est O ou S;
(b) des amides, (-CONH);
(c) des hétéroatomes choisis parmi -O-, -S- et -NR-; où R représente un groupe alkyle en $C_1$ à $C_6$; et
(d) un groupe carbonyle.

**2.** Analogue marqué selon la revendication 1, dans lequel la chaîne de liaison contient un groupe hétérocyclique choisi parmi les groupes 1,4-pipérazinylène, 2,5-diméthyl-1,4-pipérazinylène, 1,3-imidazolidinylène et 1,3-hexahydrodiazépinylène.

**3.** Analogue marqué selon la revendication 1, répondant à la structure :

(I)

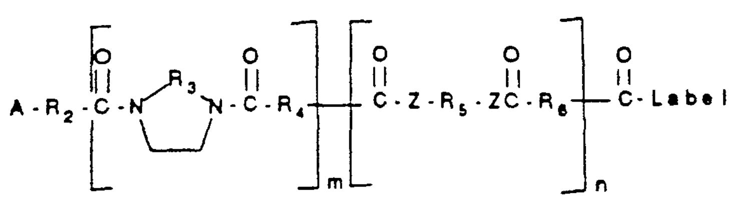

dans laquelle:

A représente un noyau hydantoïne de structure

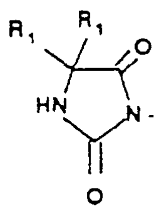

ou un noyau barbiturate de structure

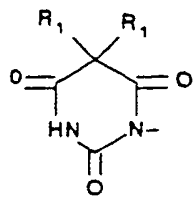

les radicaux $R^1$ représentent chacun indépendamment l'un de l'autre un atome d'hydrogène ou un groupe alkyle de 1 à 10 atomes de carbone, phényle non substitué ou substitué;
les radicaux $R^2$, $R^4$, $R^5$ et $R^6$ représentent chacun indépendamment les uns des autres un groupe alkylène en $C_1$ à $C_{10}$ ou des groupes alkylène interrompus par au moins un ou plusieurs groupes ester, groupes amide, -O-, -S- et -NR-;
$R^3$ représente un groupe alkylène en $C_1$ à $C_3$;
Z représente -O-, -S- ou -NR-, où R représente un atome d'hydrogène ou un groupe alkyle en $C_1$ à $C_6$;
m vaut 0, 1, ou 2;
n vaut 0, 1, ou 2:
$m + n > 0$; et
le nombre total d'atomes compris dans m, n et $R^2$ est de 12 à 40;
MARQUEUR est une enzyme;
et en outre à condition (i) qu'au moins un des groupes $R^1$ soit un groupe phényle substitué ou non substitué; (ii) qu'un des radicaux $R^4$, $R^5$ et $R^6$ puisse être un groupe phénylène: et (iii) que les constituants de structure I entre crochets puissent s'y trouver dans n'importe quel ordre.

**4.** Analogue marqué selon la revendication 3, dans lequel :

les radicaux $R^1$ peuvent représenter chacun indépendamment l'un de l'autre un atome d'hydrogène ou un groupe méthyle, propyle, hexyle, décyle, phényle non substitué ou phényle substitué par un groupe alkyle de 1 à 6 atomes de carbone, nitro, halogéno, cyano et alcoxy de 1 à 6 atomes de carbone;
les radicaux $R^2$, $R^4$, $R^5$ et $R^6$ représentent chacun indépendamment les uns des autres un groupe alkylène choisi parmi les groupes éthylène, triméthylène, butylène, pentylène, octylène, ou un tel groupe alkylène interrompu par au moins un ou plusieurs groupes ester, groupes amide, -O-, -S- ou -NR-;
$R^3$ représente un groupe méthylène, éthylène ou triméthylène; et
Z représente -NR-, où R représente au moins un atome d'hydrogène ou un groupe méthyle, propyle ou hexyle; et
MARQUEUR représente une enzyme.

**5.** Analogue marqué selon la revendication 1, dans lequel le marqueur est choisi parmi la peroxydase de raifort (HRP) ou une peroxydase de raifort enrichie en amine (AHRP); le noyau de médicament marqué est la phénytoïne ou le phénobarbital et le groupe de liaison est choisi parmi les groupes :

tétraméthylènecarbonyliminohexaméthylèneiminocarbonyléthylènecarbonyle,
tétraméthylènecarbonyl-1,4-pipérazinylènecarbonyléthylènecarbonyle, et
tétraméthylènecarbonyliminoéthylèneoxycarbonyléthylènecarbonyle.

**6.** Procédé de fabrication des analogues de médicament marqués selon l'une quelconque des revendications précédentes, comprenant les étapes consistant à :

(1) mettre en contact un marqueur portant un groupe amine ou sulfhydryle avec un excès d'un analogue de médicament qui comprend ;

(a) un groupe ester actif, tel que succinimidoxycarbonyle;
(b) un noyau de médicament choisi parmi un noyau hydantoïne ou un noyau barbiturate; et
(c) une chaîne de liaison qui relie le groupe ester actif au noyau hydantoïne ou barbiturate;

la chaîne de liaison étant telle que définie dans la revendication 1, et
(2) éliminer l'ester actif non utilisé et les sous-produits de condensation, de préférence par dialyse.

**7.** Procédé selon la revendication 6, dans lequel l'analogue de médicament et le marqueur sont dissous dans un solvant organique miscible à l'eau ou dans un mélange de solvant et d'eau avant leur mise en contact.

**8.** Analogue de médicament marqué selon l'une quelconque des revendications 1 à 4, dans lequel la chaîne de liaison comporte 12 à 18 atomes.

**9.** Analogue de médicament marqué selon l'une quelconque des revendications 3, 4 ou 5, dans lequel Z est -NR-.